Europäisches Patentamt

European Patent Office

Office européen des brevets

(11) Veröffentlichungsnummer: **0 113 798**
**A1**

(12) **EUROPÄISCHE PATENTANMELDUNG**

(21) Anmeldenummer: 83107190.7

(22) Anmeldetag: 22.07.83

(51) Int. Cl.³: **C 07 C 43/11**, C 07 C 43/13,
C 07 C 43/23, C 07 C 41/03,
C 08 G 63/20, C 08 G 63/46

(30) Priorität: 16.12.82 DE 3246611

(43) Veröffentlichungstag der Anmeldung: **25.07.84**
**Patentblatt 84/30**

(84) Benannte Vertragsstaaten: **AT BE CH DE FR GB IT LI NL SE**

(71) Anmelder: **Henkel Kommanditgesellschaft auf Aktien,
Postfach 1100 Henkelstrasse 67,
D-4000 Düsseldorf-Holthausen (DE)**

(72) Erfinder: **Meffert, Alfred, Dr., Marie-Curie-Strasse 10,
D-4019 Monheim (DE)**
Erfinder: **Wilk, Hans-Christoph, Dr., An der Obererft 94,
D-4040 Neuss (DE)**

(54) Verfahren zur Herstellung von Alkoholgemischen aus epoxidierten Fettalkoholen.

㊗ Die Erfindung betrifft neue überwiegend oleochemische Rohstoffe mit zahlreichen Hydroxylgruppen. Beschrieben werden die Umsetzungsprodukte epoxidierter Fettalkohole mit mehrfunktionellen Alkoholen und/oder Phenolen sowie ein Verfahren zur Gewinnung dieser Stoffe, welches je nach Ausgangsmolverhältnis Alkoholgemische mit überwiegend primären bzw. überwiegend sekundären Hydroxylgruppen liefert. Weiterhin werden die Umsetzungsprodukte der Alkoholgemische mit cyclischen Ethern wie Ethylenoxid oder Propylenoxid beschrieben.

EP 0 113 798 A1

Erfinder: Dr. Wilk
Dr. Meffert

0113798
HENKEL KGaA
ZR-FE/Patente
Dr.Wik/Po/Br/
Bö/Re

Henkelstr. 67
4ooo Düsseldorf, den 07.12.1982

Patentanmeldung

D 6651 EP

"Verfahren zur Herstellung von Alkoholgemischen aus epoxidierten Fettalkoholen"

Die Erfindung liegt auf dem Gebiet organischer Produkte, welche sich von nachwachsenden Rohstoffen ableiten. Sie betrifft ein Verfahren zur Herstellung neuer Alkoholgemische durch Umsetzung von Fettalkoholen mit mehrfunktionellen Hydroxyverbindungen. Die neuen Alkoholgemische können zur Herstellung von Polymeren, beispielsweise Alkydharzen, verwendet werden.

Es sind bereits Verfahren bekannt, Epoxyalkane mit mehr als 8 Kohlenstoffatomen, aber keiner weiteren funktionellen Gruppe mit Alkoholen umzusetzen. So wird in der US-PS 34 75 499 ein Verfahren beschrieben, bei dem Temperaturen zwischen $20^{\circ}$ und $200^{\circ}C$ angewendet werden und Katalysatoren, wie z.B. Alkalimetallhydroxide, z.B. NaOH KOH oder auch Säuren wie $H_2SO_4$ oder HCL, zugegen sind. Weiterhin wird in der US-Patentschrift 36 07 778 die Verwendung von Bortrifluoridetherat als Katalysator beschrieben.

Ein hinsichtlich der erreichbaren Produktqualität - hellfarbige Produkte werden angestrebt - verbessertes Verfahren zur Ringöffnung von Epoxyalkanen mit Alkoholen ist in der deutschen Patentanmeldung 29 00 030 beschrieben. Danach werden Epoxyalkane mit entständiger oder innenständiger Epoxidgruppe und 6 - 18 Kohlenstoffatomen und mono- oder polyfunktionelle Alkohole, die 1 - 10 Kohlenstoffatome und 1 - 4 alkoholische Hydroylgruppen enthalten, in einem Molverhältnis von 1 : 1,1 bis 1 : 10 in Abwesenheit

...

- 2 -

von Lösungsmittel und in Gegenwart von Schwefelsäure oder aromatischen Sulfonsäuren mit höchstens 8 Kohlenstoffatomen bei 50 bis 130°C miteinander umgesetzt. Nach Neutralisation der Säure werden die Umsetzungsprodukte durch Abdestillieren flüchtiger Bestandteile bei Temperaturen bis höchstens 150° unter vermindertem Druck angereichert.

Nach den genannten Verfahren lassen sich Gemische mehrfunktioneller Alkohole herstellen. Doch weisen die so erhaltenen Polyole im Vergleich zu den Ausgangsverbindungen einen nur um 3 Atome vergrößerten Abstand der Hydroxylgruppen auf. Für viele Anwendungen - z.B. für die Herstellung flexibler Polyester - ist es jedoch erwünscht, Polyole mit einem möglichst großen Abstand der Hydroxylgruppen im Molekül zur Verfügung zu haben. Außerdem sind Olefinoxide petrochemische, also nicht nachwachsende Rohstoffe.

Es ist somit Aufgabe der Erfindung, neue Alkoholgemische zur Verfügung zu stellen, die zumindest überwiegend aus nachwachsenden Rohstoffen gewonnen werden können. Eine weitere Aufgabe der Erfindung ist es, Alkohole bereitzustellen, die einen Abstand von mindestens 10 Atomen zwischen 2 Hydroxylgruppen aufweisen.

Die erfindungsgemäße Aufgabe wurde gelöst durch ein Verfahren zur Herstellung von Alkoholgemischen, die insbesondere für die Alkydharzsynthese geeignet sind, enthaltend Polyole mit mehr als 10 C-Atomen und mehr als 2 Hydroxylgruppen im Molekül, dadurch gekennzeichnet, daß man epoxidierte $C_{12}$-$C_{26}$-Fettalkohole mit

...

2 - 10 OH-Gruppen enthaltenden aliphatischen und/oder aromatischen Polyolen unter Öffnung der Epoxidringe umsetzt und gewünschtenfalls das Reaktionsprodukt nachfolgend mit $C_2$-$C_4$-Epoxiden zur Reaktion bringt.

Die in Rede stehenden Alkoholgemische sind chemische Verbindungen, die sich überwiegend von nachwachsenden Rohstoffen ableiten. Grundlage sind Triglyceride, also fette Öle, pflanzlicher, tierischer oder seetierischer Herkunft. Es ist bekannt, diese z.B. zu Methylestern umzusetzen, welche sich katalytisch zu Alkoholen, den sogenannten Fettalkoholen, reduzieren lassen. Unter Fettalkoholen versteht man primäre monofunktionelle Alkohole, welche sich von Fettsäurederivaten ableiten. Die technisch zugänglichen Fettalkohole sind keine reinen chemischen Substanzen, sondern liegen als Gemische unterschiedlicher Kettenlängen vor und weisen zwischen 0 und 3 Doppelbindungen auf. Die Zusammensetzung der Fettalkoholgemische ist abhängig von der Art des Triglycerids aus dem sie gewonnen worden sind. Es ist jedoch möglich, durch Extraktion, fraktionierte Kristallisation oder Destillation in den Gemischen einzelne Fettalkohole anzureichern oder Fettalkohole aus bereits derartig vorbehandelten Fettsäure-Fraktionen herzustellen.

Im folgenden sei unter dem Begriff Fettalkohol stets eine Mischung mehrerer meist gesättigter und ungesättigter Alkohole verstanden. Die am weitesten verbreiteten ungesättigten Bestandteile von Fettalkoholen sind:

$C_{16}$ : Palmitoleylalkohol

$C_{18}$ : Oleylalkohol, Elaidylalkohol, Linoleylalkohol, Linolenylalkohol

$C_{20}$ : Gadoleylalkohol, Arachedonalkohol

$C_{22}$ : Erucaalkohol, Brasidylalkohol

Fettalkohole, die einen dieser Bestandteile in großen Mengen oder überwiegen enthalten, können nach bekannten Methoden etwa mit Hilfe von Persäuren wie Peressigsäure, epoxidiert werden. Die Epoxidation einfach ungesättigter Fettalkohole läßt sich mit hohen Umsätzen, etwa 80 - 100 mol-% Umsatz durchführen. Bei mehrfach ungesättigten Alkoholen wird nur die erste Doppelbindung quantitativ epoxidiert. Die Epoxidation weiterer Doppelbindungen erfordert drastische Reaktionsbedingungen.

Zur Herstellung der erfindungsgemäßen polyolhaltigen Gemische werden epoxidierte Fettalkohole verwendet, die 30 - 100 mol-% Epoxidgruppen - vorzugsweise 70 - 95 mol-% Epoxidgruppen - bezogen auf ursprünglich vorhandene Doppelbindungen aufweisen. Verwendet werden können epoxidierte Fettalkohole auf Basis von Fettalkoholen mit Iod-Zahlen zwischen 20 und 170. Geeignete epoxidierte Fettalkohole leiten sich zum Beispiel von folgenden Fettalkoholen ab:

Talgalkohol mit Iod-Zahl 50 - 55, enthaltend gesättigte Alkohole $C_{12}$ - $C_{20}$, hergestellt aus Talgfettsäureestern, Talgalkohol mit Iod-Zahl 80 - 85, hergestellt aus Talgfettsäureestern, deren ungesättigte Anteile nach dem Umnetzverfahren angereichert worden sind.

. . .

0113798
HENKEL KGaA
ZR-FE/Patente

Talgalkohol mit Iod-Zahl 92 - 96, hergestellt aus Talgalkohol mit Iod-Zahl 80 - 85, durch Auskristallisieren
und Abtrennen gesättigter Anteile. Das Produkt besteht
zu ca. 90 % aus Oleylalkohol.

Sojaalkohol mit Iod-Zahl 110 - 130, hergestellt aus
Soja-Fett-Säure, enthaltend ca. 30 % Oleylalkohol, ca.
40 % Linoleylalkohol und ca. 7 % Linolenylalkohol.

Rübölalkohol mit Iod-Zahl 90 - 100, enthaltend ca.
50 Gew.-% Erucaalkohol.

Weiterhin von Fettalkoholen auf Basis von Tranfettsäure,
Palmölfettsäure, Leinölfettsäure, Erdnußölfettsäure,
Baumwollölfettsäure.

Geht man von einem Fettalkohol mit Iod-Zahl zwischen
50 und 55 aus, so wird nach Epoxidation eine Epoxidzahl von 2,5 - 2,7 Gew.-% Epoxidsauerstoff erreicht.

Fettalkohole mit Iod-Zahlen zwischen 110 und 130 lassen sich bis zu einer Epoxidzahl von ca. 5 Gew.-%
Epoxidsauerstoff epoxidieren. Es verbleibt eine Restiodzahl zwischen 2 und 30.

Erfindungsgemäß werden die epoxidierten Fettalkohole
der katalytischen Ringöffnung mit mehrfunktionellen
Hydroxyverbindungen unterworfen. Zur sauer katalysierten Ringöffnung legt man zweckmäßigerweise die mehrfunktionelle Hydroxykomponente und katalytische Mengen
einer Säure, etwa Schwefelsäure, Phosphorsäure, 4-To-
luolsulfonsäure oder saureIonenaustausche im Reaktionsgefäß vor und gibt bei Temperaturen zwischen
50 und 130°C, vorzugsweise zwischen 70 und 100°C
epoxidierten Fettalkohol portionsweise zu.

. . .

BAD ORIGINAL

Der Reaktionsverlauf läßt sich gaschromotographisch oder aber über Titration des Restepoxidsauerstoffs bestimmen. Nach Reaktionszeiten von 2 bis 6 Stunden, vorzugsweise von 3 bis 5 Stunden, je nach Ansatzgröße und Zulaufgeschwindigkeit, ist der epoxidierte Fettalkohol quantitativ umgesetzt. Am Ende der Reaktionszeit kann der saure Katalysator durch geeignete Basen neutralisiert werden. Je nach Verwendungszweck der polyolhaltigen Gemische können leicht flüchtige Anteile, wie z.B. nicht umgesetzte mehrfunktionelle Hydroxyverbindungen oder auch gesättigte oder nicht-gesättigte nichtepoxidierte Fettalkohole im Vakuum abdestilliert werden.

Die saure Katalyse ist bei der Umsetzung der epoxidierten Fettalkohole mit mehrfunktionellen Alkoholen bevorzugt. Zur Durchführung der sauer katalysierten Ringöffnung sind weitere Verfahrensvarianten geeignet. So können alle Ausgangsstoffe gleichzeitig in das Reaktionsgefäß gegeben werden, oder auch die mehrfunktionellen Hydroxykomponente oder der Fettalkohol zudosiert werden.

Die Ringöffnung epoxidierter Fettalkohole mit Phenolen wird vorzugsweise basisch katalysiert. Geeignete Basen sind z.B. Alkoholate oder Phenolate von Alkalimetallen, oder tertiäre Amine. Die basisch katalysierte Umsetzung findet bei Temperaturen zwischen 100 und 180°, vorzugsweise 120 - 160°C, statt. Es ist hier bevorzugt, alle Reaktionskomponenten gleichzeitig in das Reaktionsgefäß zu geben, doch können auch die Phenole oder aber die epoxidierten Fettalkohole vorgelegt werden. Allgemein sind die Reaktionszeiten hier etwas länger als bei der sauren Katalyse. So findet vollständiger Umsatz z.B. nach 4 - 8 Standen statt.

. . .

Nach Reaktionsende können die Katalysatoren durch Säurezugabe zerstört werden. Für viele Anwendungen ist es zweckmäßig, die Reaktionsprodukte salzfrei zu waschen und/oder flüchtige Bestandteile im Vakuum abzuziehen.

Die erfindungsgemäßen polyolhaltigen Gemische enthalten je nach Menge der eingesetzten Reaktanten und nach Art der Zudosierung Moleküle unterschiedlicher Struktur. So entsteht bei Zugabe der epoxidierten Fettalkohole zu einem Überschuß von mehrfunktionellen Hydroxyverbindungen in erster Linie das 1:1-Addukt. Das 1 : 1 - Addukt ist ein Dialkylether (oder Alkylphenylether), dessen einer Alkylrest dem epoxydierten Fettalkohol entstammt und in $\beta$ -Stellung zur Ethergruppe eine sekundäre Hydroxylgruppe aufweist. Der andere Alkylrest (oder Phenylrest) entstammt der mehrfunktionellen Hydroxyverbindung. Er trägt n -1 Hydroxylgruppen, wenn eine mehrfunktionelle Hydroxyverbindung mit n-Hydroxylgruppen eingesetzt worden ist.

Befindet sich in einer Reaktionsmischung das entstandene 1 : 1 - Addukt neben unumgesetzter mehrfunktioneller Hydroxyverbindung, so kann der Epoxidring eines weiteren Moleküls epoxidierten Fettalkohols einerseits durch die Umsetzung mit der mehrfunktionellen Hydroxyverbindung zu einem weiterem Molekül 1 : 1 - Addukt geöffnet werden, andererseits kann jedoch auch eine Reaktion mit einer Hydroxylgruppe des 1 : 1 - Adduktes zum 2 : 1 - Addukt stattfinden. Bei dieser Reaktion sind 2 Arten von 2 : 1 - Addukten denkbar, nämlich solche, bei denen die aus dem Fettalkohol stammende Hydroxylgruppe des 1 : 1 - Addukts mit einer weiteren Epoxidgruppe reagiert und solche, bei denen die Hydroxylgruppe der Hydroxyverbindung des 1 : 1 - Addukts mit einer weiteren Epoxidgruppe reagiert.

. . .

Sowohl polyolhaltige Gemische, die in erster Linie 1 : 1-Addukte als auch solche, die in erster Linie 2 : 1 - Addukte enthalten, sind wertvolle Rohstoffe zur Herstellung von Alkydharzen. Um in erster Linie 1 : 1 - Addukte zu erhalten, ist es zweckmäßig, die mehrfunktionelle Hydroxykomponente im Überschuß, etwa in einem 3-molaren oder auch 10- molaren Überschuß einzusetzen. Es empfiehlt sich weiterhin, epoxidierten Fettalkohol während der Umsetzung in mehreren Portionen oder kontinuierlich zuzugeben. Zur Herstellung von 2 : 1 - Addukten oder noch höheren Addukten werden 2 oder mehr mol epoxydierter Fettalkohol pro mol mehrfunktionelle Hydroxyverbindung eingesetzt.

Verwendet man hierbei epoxidierte Fettalkohole, die noch einen gewissen Anteil an gesättigten Fettalkoholen enthalten oder gibt man gesättigte oder ungesättigte Fettalkohole zu den epoxidierten Fettalkoholen zu, so findet auch Reaktion der Epoxidgruppen mit diesen Fettalkoholen statt, was für viele Anwendungen gewünscht sein kann.

Zur Ringöffnung der epoxidierten Fettalkohole kann eine Vielzahl von mehrfunktionellen hydroxylgruppenhaltigen Verbindungen eingesetzt werden. Einzige Voraussetzung ist, daß zwischen 2 und 10 Hydroxylgruppen pro Molekül vorliegen. Diese können primär, sekundär oder auch tertiär sein, aliphatisch oder aromatisch. Bevorzugt ist die Umsetzung mit solchen Hydroxylverbindungen, die bereits zur Herstellung von Polyestern oder Polyurethanen verwendet werden und demzufolge in technischen Mengen verfügbar sind. So sind z.B. unter den Dihydroxyverbindungen bevorzugt: Ethylenglykol, Propylenglykol, die isomeren Butandiole, Neopentylglykol, Hexandiol sowie die aus diesen Verbindungen durch Selbstkondensation

...

- 9 -

herstellbaren Oligomeren wie z.B. Di-, Tri- und Tetraglykole.

Unter den aliphatischen trifunktionellen Hydroxyverbindung sind Glycerin, Trimethylolethan und Trimethylol-propan bevorzugt. Bevorzugte aliphatische Hydroxyver-bindungen mit mehr als 3 OH-Gruppen sind Pentaerythrit, Sorbit sowie die Selbstkondensationsprodukte des Glycerins mit Kondensationsgrad von 2 - 5. Weitere ge-eignete mehrfunktionelle Hydroxyverbindungen sind Gly-kole, welche durch Ringöffnung längerkettiger oder cyclischer Epoxide mit Wasser hergestellt werden können. So sind beispielsweise 1,2-Dihydroxyalkane geeignet. Weiter geeignet sind aus epoxidierten Terpenen hergestellte Glykole, z.B. Glykole aus epoxidierten Limonen.

Als aromatische mehrfunktionelle Hydroxyverbindungen sind ein- oder mehrkernige Phenole mit 2 und mehr Hydroxylgruppen im Molekül geeignet. Bevorzugt sind Hydrochinon, Resorcin sowie mehrkernige Phenole vom Typ des Bisphenol A.

Die erfindungsgemäßen polyolhaltigen Gemische können in einer zweiten Reaktionsstufe mit kurzkettigen Epoxiden wie z.B. Ethylenoxid, Propylenoxid, Butenoxid, Isobutenoxid, Glycid oder Epichlorhydrin weiter umgesetzt werden. Es tritt dadurch eine Kettenverlängerung an den Hydroxylgruppen auf bzw. eine Vergrößerung der Anzahl der Hydroxylgruppen im Falle der Verwendung von Glycid. Die kurzkettigen Epoxide, welche 2 bis 4 Kohlenstoffatome aufweisen, werden in Mengen zwischen einem und 40 mol pro mol polyolhaltiges Gemisch eingesetzt.

...

Verfahrenstechnisch wird hier bei der basischen Katalyse vorzugsweise so vorgegangen, daß die Katalysatoren am Ende der Umsetzung der Hydroxyverbindungen mit den epoxidierten Fettalkoholen nicht durch Neutralisation zerstört werden. Bei der sauren Katalyse wird mit Basen, z.B. Alkalimetallalkoholaten überneutralisiert. Es werden dann die Epoxide mit 2-4 Kohlenstoffatomen zugegeben und unter üblichen Bedingungen, d.h. unter Druck bei Temperaturen zwischen 100 und 200°C mit den polyolhaltigen Gemischen umgesetzt.

Nach einer bevorzugten Arbeitsweise werden zunächst Umsetzungsprodukte von Glycerin, Trimethylolethan, Trimethylolpropan und/oder Pentaerythrit mit Propylenoxid, Ethylenoxid oder anderen 2 - 4 C-Atomen enthaltenden Epoxiden im Molverhältnis 1 : 1 bis 1 : 30 hergestellt. Die so erhaltenen Polyole können zur Ringöffnung der epoxidierten Fettalkohole verwendet werden.

Die erfindungsgemäßen Alkoholgemische sind wertvolle Rohstoffe auf - zumindest überwiegend - oleochemischer d.h. natürlicher, nachwachsender Basis. Sie sind zur Herstellung von Polykondensaten, insbesondere zur Herstellung von Alkydharzen geeignet.

Lack, insbesondere Wasserlacke auf Basis erfindungsgemäßer Alkoholgemische zeigen unerwartet gute Eigenschaften vor allem bezüglich Glanz und Glanzhaltung bei Bewitterung.

...

Patentanmeldung D 6651 EP - 11 -

HENKEL KGaA
ZR-FE/Patente

0113798

Beispiele

## 1. Epoxidierte Fettalkohole

Tabelle 1

Kennzahlen der epoxidierten Fettalkohole

| Nr. | Epoxidzahl (mol % Epoxidsauerstoff) | Zusammensetzung $C_{10}$ $C_{12}$ $C_{14}$ $C_{16}$ $C_{18}$ $C_{20}$ | | | | | | Hydroxylzahl mg KOH/g | Ausgangsjodzahl mg J/g |
|---|---|---|---|---|---|---|---|---|---|
| A | 2,5 - 2,7 | 0 | 0-2 | 2-7 | 25-35 | 55-75 | 0-2 | 210 - 220 | 50 - 55 |
| B | 4,1 - 4,3 | 0 | 0-2 | 2-7 | 8-18 | 70-83 | 0-3 | 209 - 210 | 84 - 89 |
| C | 4,4 - 4,7 | 0 | 0 | 0 | 2-9 | 90-97 | 0-2 | 200 - 210 | 92 - 96 |
| D | 4,3 - 4,8 | 0 | 0 | 0 | 5-10 | 90-95 | 0-2 | 200 - 220 | 110 - 130 |

Die epoxidierten Fettalkohole können nach bekannten Methoden hergestellt werden, z.B. nach der Methode von D. Swern et al., J. Am. Chem. Soc. 66, 1925 (1944).

2. Herstellung der polyolhaltigen Gemische.

2.1 Sauer katalysierte Umsetzung: Die hydroxylgruppenhaltige Verbindung wir vorgelegt und mit 0,1 bis 0,5 g Schwefelsäure pro mol umzusetzendes Epoxid versetzt. Man heizt auf 70 °C bis 100 °C auf und gibt den epoxierten Fettalkohol in geschmolzenem Zustand zu. Das Reaktionsende (bei 1 kg Laboransätzen nach 3 Stunden) läßt sich gaschromatographisch bestimmen. Man neutralisiert die Schwefelsäure mit Natriummethylat und destilliert gewünschtenfalls nicht umgesetzte Alkohole im Vakuum ab. Die Umsetzung der Epoxidgruppen ist quantitativ.

Kennzahlen der so erhaltenen polyolhaltigen Gemische sind in Tab. 2 aufgelistet.

Tabelle 2

Kennzahlen der Alkoholgemische (sauer katalysierte Ringöffnung).

| Nr. | Epox.Fettalkohol | Hydroxy-Verbindung | Molverhältnis Epox.Fettalkohol | OH-Zahl (mg KOH/g) |
|---|---|---|---|---|
| 1 | A | Glycol | 1:3 | 394,0 |
| 2 | A | Glycol | 1:1 | 328,4 |
| 3 | A | Glycol | 2:1 | 286,7 |
| 4 | C | Glycol | 1:3 | 407,7 |
| 5 | C | Glycol | 1:1 | 347,4 |
| 6 | C | Glycol | 2:1 | 297,4 |

| Nr. | | | Molverhältnis | OH-Zahl |
|---|---|---|---|---|
| 7 | C | Glycerin | 1:3 | 411,4 |
| 8 | C | Glycerin | 1:3 | 364,5 |
| 9 | C | Trimethylol-propan | 1:3 | 472,7 |
| 10 | C | dito | 3:1 | 276,3 |
| 11 | D | Glycol | 1:3 | 410,0 |
| 12 | D | Glycol | 1:2 | 394,5 |
| 13 | D | Glycol | 1:1 | 359,7 |

2.2    Basisch katalysierte Umsetzung.

Epoxidierter Fettalkohol und Phenol, z.B. Bisphenol A werden im gewünschten Molverhältnis gemischt und mit 1 Gewichtsprozent Natriummethylat, bezogen auf umzusetzenden epoxidierten Fettalkohol, versetzt. Es wird ca. 6 Stunden bei 120-160 $^{o}$C umgesetzt und vorzugsweise nach Verdünnen mit einem Lösungsmittel wie z.B. Toluol mit heißem Wasser salzfrei gewaschen. Anschließend wird das Lösungsmittel durch Destillation entfernt. Kennzahlen der so erhaltenen Alkoholgemische sind in Tab. 3 aufgelistet.

Tabelle 3

Alkoholgemische hergestellt durch basisch katalysierte Umsetzung epoxidierter Fettalkohole mit Bisphenol 4.

| Nr. | Epox.Fettalkohol | Molverhältnis Epox.Fettalkoholen Bisphenol A | OH-Zahl (mg KOH/g) |
|---|---|---|---|
| 14 | A | 2:1 | 295,6 |
| 15 | A | 1:1 | 327,9 |
| 16 | C | 3:1 | 30o,8 |
| 17 | C | 2:1 | 30y,8 |
| 18 | C | 1:1 | 341,7 |
| 19 | D | 2:1 | 325,3. |

2.3 Umsetzungsprodukte der durch Ringöffnung epoxidierter Fettalkohole mit Hydroxyverbindungen erhalten Alkoholgemische mit Ethylenoxid oder Propylenoxid.

Die Ringöffnung der epoxidierten Fettalkohole wurde wie unter 2.1 und 2.2 beschrieben durchgeführt, doch wurden die basischen Katalysatoren am Reaktionsende nicht durch Neutralisation zerstört. Die sauren Katalysatoren wurden überneutralisiert. Es wurde dann in einem Autoklaven die gewünschte Menge Ethylenoxid bzw. Propylenoxid zugegeben und bei 160 bzw. 180°C umgesetzt. Der Reaktionsverlauf ließ sich anhand der Druckentwicklung kontrollieren. Am Ende der Umsetzung wurden die Katalysatoren durch Neutralisation zerstört. Umsetzungsprodukte auf Basis Bisphenol A wurden salzfrei gewaschen. Die OH-Zahlen der Produkte sind in Tabelle 4 aufgeführt.

Tabelle 4

Umsetzungsprodukte der polyolhaltigen Gemische mit Ethylenoxid / Propylenoxid

| Nr. | Epox: Fettalkohol | Hydroxyverbindung | Molverhältnis epox. Fettalkohol zu Hydroxyverbindg. | mol Ethylenoxid pro mol Polyol | mol Propylenoxid pro mol Polyol | OH-Zahl (mg KOH/g) |
|---|---|---|---|---|---|---|
| 20 | A | Glycol | 1 : 3 | 2 | | 324,4 |
| 21 | A | " | 1 : 3 | 5 | | 257,1 |
| 22 | A | " | 1 : 3 | | 3 | 311,4 |
| 23 | A | " | 1 : 3 | | 6 | 241,7 |
| 24 | A | " | 1 : 1 | 3 | | 258,1 |
| 25 | A | " | 1 : 1 | 6 | | 213,8 |
| 26 | A | " | 1 : 1 | | 3 | 243,2 |
| 27 | A | " | 1 : 1 | | 6 | 207,6 |
| 28 | A | " | 2 : 1 | 3 | | 229,1 |
| 29 | A | " | 2 : 1 | 6 | | 193,8 |
| 30 | A | " | 2 : 1 | | 2 | 241,6 |
| 31 | A | " | 2 : 1 | | 4 | 211,7 |
| 32 | A | Bisphenol-A | 2 : 1 | 2 | | 254,3 |
| 33 | A | " | 2 : 1 | 5 | | 216,7 |

Patentanmeldung D 6651 EP - 15 -

HENKEL KGaA
ZR-FE/Patente

0113798

Tabelle 4 (Fortsetzung)

| Nr. | Epox: Fettalkohol | Hy droxyverbindung | Molverhältnis epox. Fettalkohol zu Hydroxyverbindg. | mol Ethylenoxid pro mol Polyol | mol Propylenoxid pro mol Polyol | OH-Zahl (mg KOH/g) |
|---|---|---|---|---|---|---|
| 34 | A | Bisphenol-A | 2 : 1 | | 3 | 230,1 |
| 35 | A | " | 2 : 1 | | 6 | 191,7 |
| 36 | A | " | 1 : 1 | 3 | | 260,2 |
| 37 | A | " | 1 : 1 | 6 | 3 | 214,1 |
| 38 | A | " | 1 : 1 | | 6 | 249,1 |
| 39 | A | " | 1 : 1 | | | 201,3 |
| 40 | B | Glycol | 1 : 3 | 1 | | 338,7 |
| 41 | B | " | 1 : 3 | 3 | | 287,7 |
| 42 | B | " | 1 : 3 | | 2 | 295,8 |
| 43 | B | " | 1 : 3 | | 4 | 245,7 |
| 44 | B | " | 1 : 1 | 5 | | 248,3 |
| 45 | B | " | 1 : 1 | 3 | | 275,0 |
| 46 | B | " | 1 : 1 | | 2 | 280,6 |
| 47 | B | " | 1 : 1 | | 4 | 245 |
| 48 | B | " | 2 : 1 | 3 | | 248,5 |
| 49 | B | " | 2 : 1 | 5 | | 226,5 |
| 50 | B | " | 2 : 1 | | 2 | 253,1 |

Patentanmeldung D 6651 EP

- 16 -

HENKEL KGaA
ZR-FE/Patente

0113798

## Tabelle 4 (Fortsetzung)

| Nr. | Epox: Fettalkohol | Hy droxyver-bindung | Molverhältnis epox. Fettalko-hol zu Hy-droxyverbindg. | mol Ethylen-oxid pro mol Polyol | mol Propy-lenoxid pro mol Polyol | OH-Zahl (mg KOH/g) |
|---|---|---|---|---|---|---|
| 51 | B | Glycol | 2 : 1 | | 4 | 223,8 |
| 52 | B | Bisphenol-A | 2 : 1 | 2 | | 277,8 |
| 53 | B | " | 2 : 1 | 4 | | 250,7 |
| 54 | B | " | 2 : 1 | | 2 | 270,6 |
| 55 | B | " | 2 : 1 | | 4 | 242,4 |
| 56 | B | " | 1 : 1 | 2 | | 275,9 |
| 57 | B | " | 1 : 1 | 4 | | 240,3 |
| 58 | B | " | 1 : 1 | | 2 | 271,8 |
| 59 | B | " | 1 : 1 | | 4 | 236,7 |
| 60 | C | Glycol | 1 : 3 | 2 | | 334,5 |
| 61 | C | " | 1 : 3 | 5 | | 262,0 |
| 62 | C | " | 1 : 3 | | 2 | 322,2 |
| 63 | C | " | 1 : 3 | | 5 | 239,5 |
| 64 | C | " | 1 : 1 | 3 | | 269,2 |
| 65 | C | " | 1 : 1 | 6 | | 220,3 |
| 66 | C | " | 1 : 1 | | 2 | 287,1 |
| 67 | C | " | 1 : 1 | | 4 | 241,7 |

## Tabelle 4 (Fortsetzung)

| Nr. | Epox:<br>Fettalkohol | Hy droxyver-<br>bindung | Molverhältnis<br>epox. Fettalko-<br>hol zu Hy-<br>droxyverbindg. | mol Ethylen-<br>oxid pro mol<br>Polyol | mol Propy-<br>lenoxid pro<br>mol Polyol | OH-Zahl<br>(mg KOH/g) |
|---|---|---|---|---|---|---|
| 68 | C | Glycol | 2 : 1 | 3 | | 239,9 |
| 69 | C | " | 2 : 1 | 6 | | 200,6 |
| 70 | C | " | 2 : 1 | | 2 | 250,2 |
| 71 | C | " | 2 : 1 | | 4 | 216,5 |
| 72 | C | Glycerin | 1 : 3 | 3 | | 327,9 |
| 73 | C | " | 1 : 3 | 6 | | 270,8 |
| 74 | C | " | 1 : 3 | | 3 | 312,2 |
| 75 | C | " | 1 : 3 | | 6 | 259,7 |
| 76 | C | " | 1 : 1 | 3 | | 296,7 |
| 77 | C | " | 1 : 1 | 6 | | 249,3 |
| 78 | C | " | 1 : 1 | | 2 | 309,9 |
| 79 | C | " | 1 : 1 | | 4 | 266,2 |
| 80 | C | Trimethylol-<br>propan | 1 : 3 | 2 | | 396,5 |
| 81 | C | | 1 : 3 | 4 | | 342,7 |
| 82 | C | " | 1 : 3 | | 2 | 381,9 |
| 83 | C | " | 1 : 3 | | 4 | 319,3 |
| 84 | C | Bisphenol-A | 2 : 1 | 3 | | 249,0 |

Patentanmeldung D 6651 EP — 18 —

HENKEL KGaA
ZR-FE/Patente

0113798

## Tabelle 4 (Fortsetzung)

| Nr. | Epox: Fettalkohol | Hy droxyver-bindung | Molverhältnis epox. Fettalko-hol zu Hy-droxyverbindg. | mol Ethylen-oxid pro mol Polyol | mol Propy-lenoxid pro mol Polyol | OH-Zahl (mg KOH/g) |
|---|---|---|---|---|---|---|
| 85 | C | Bisphenol-A | 2 : 1 | 6 | | 207,2 |
| 86 | C | " | 2 : 1 | | 3 | 259,6 |
| 87 | C | " | 2 : 1 | | 6 | 205,0 |
| 88 | D | " | 1 : 3 | 3 | | 308,7 |
| 89 | D | Glycol | 1 : 3 | 6 | | 246,3 |
| 90 | D | " | 1 : 3 | | 2 | 320,6 |
| 91 | D | " | 1 : 3 | | 4 | 265,1 |
| 92 | D | " | 1 : 1 | 3 | | 300,5 |
| 93 | D | " | 1 : 1 | 6 | | 240,9 |
| 94 | D | " | 1 : 1 | | 2 | 310,9 |
| 95 | D | " | 1 : 1 | | 4 | 262,1 |
| 96 | D | " | 2 : 1 | 2 | | 302,1 |
| 97 | D | " | 2 : 1 | 6 | | 228,3 |
| 98 | D | " | 2 : 1 | | 2 | 290,0 |
| 99 | D | " | 2 : 1 | | 4 | 244,7 |

## Patentansprüche

1. Verfahren zur Herstellung von Alkoholgemischen, die insbesondere für die Alkydharzsynthese geeignet sind, enthaltend Polyole mit mehr als 10 C-Atomen und mehr als 2 Hydroxylgruppen im Molekül, dadurch gekennzeichnet, daß man epoxidierte $C_{12}$ – $C_{26}$-Fettalkohole mit 2 – 10 OH-Gruppen enthaltenden aliphatischen und/oder aromatischen Polyolen unter Öffnung der Epoxidringe umsetzt und gewünschtenfalls das Reaktionsprodukt nachfolgend mit $C_2$ – $C_4$ – Epoxiden zur Reaktion bringt.

2. Verfahren nach Anspruch 1, dadurch gekennzeichnet, daß man als epoxidierte Fettalkohole Stoffgemische einsetzt, zu deren Herstellung wenigstens einer der folgenden ungesättigten Alkohole epoxidiert worden ist: Palmitoleylalkohol, Oleylalkohol, Elaidylalkohol, Linoleylalkohol, Linolenylalkohol, Gadoleylalkohol, Arachedonalkohol, Erucaalkohol oder Brasidylalkohol.

3. Verfahren nach den Ansprüchen 1 – 2, dadurch gekennzeichnet, daß epoxidierte Fettalkohole eingesetzt werden, die 30 – 100 mol-%, vorzugsweise 70 – 95 mol-%, Epoxidgruppen – bezogen auf ursprünglich vorhandene Doppelbindungen – aufweisen.

4. Verfahren nach den Ansprüchen 1 – 3, dadurch gekennzeichnet, daß als epoxidierte Fettalkoholkomponente ein Alkoholgemisch eingesetzt wird, das durch Epoxidation ungesättigter Fettalkohole auf natürlicher Basis gegebenenfalls in Abmischung mit gesättigten Fettalkoholen hergestellt worden ist.

...

5. Verfahren nach den Ansprüchen 1 - 4, dadurch gekennzeichnet, daß die epoxidierten Fettalkohole mit aliphatischen und/oder aromatischen Hydroxyverbindungen mit 2 - 4 Hydroxylgruppen umgesetzt werden.

6. Verfahren nach Anspruch 5, dadurch gekennzeichnet, daß die epoxidierten Fettalkohole mit mehrfunktionellen Alkoholen umgesetzt werden, die durch Umsetzung mit trifunktionellen und/oder tetrafunktionellen Alkoholen mit Ethylenoxid, Propylenoxid oder Glycid im Molverhältnis 1 : 1 bis 1 : 30 hergestellt worden sind.

7. Verfahren nach den Ansprüchen 1 - 4, dadurch gekennzeichnet, daß die epoxidierten Fettalkohole mit einem Kondensationsprodukt von Glycerin mit 1 - 5 Ethergruppen umgesetzt werden.

8. Verfahren nach den Ansprüchen 1 - 7, dadurch gekennzeichnet, daß die epoxidierten Fettalkohole mit den Hydroxyverbindungen innerhalb des Molverhältnisses 1 : 1 - 10, vorzugsweise innerhalb des Molverhältnisses 1 : 1,1 - 2 oder 1 : 3 - 6 umgesetzt werden.

9. Verfahren nach den Ansprüchen 1 - 8, dadurch gekennzeichnet, daß aus den Reaktionsprodukten nach der Ringöffnung durch Erhitzen auf Temperaturen bis 150° und Absenken des Drucks mit 0,1 bar leicht flüchtige Bestandteile destillativ entfernt werden.

10. Verfahren nach den Ansprüchen 1 - 9, dadurch gekennzeichnet, daß die Reaktionsprodukte der Ringöffnung nachfolgend mit Epoxiden mit 2 - 4 C-Atomen im Verhältnis 1 : 1 bis 1 : 40 umgesetzt werden.

. . .

- 22 -

11. Alkoholgemische, insbesondere für die Alkydharzsynthese, hergestellt nach einem der Verfahren 1 - 10.

12. Verwendung der Alkoholgemische nach einem der Ansprüche 1 - 11 als Rohstoffe zur Herstellung von Polyestern, insbesondere von Alkydharzen.

Europäisches
Patentamt

**EUROPÄISCHER RECHERCHENBERICHT**

0113798

Nummer der Anmeldung

EP 83 10 7190

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | FR-A-1 423 346 (BRITISH PETROLEUM) * ganzes Dokument * | 1-12 | C 07 C 43/11<br>C 07 C 43/13<br>C 07 C 43/23<br>C 07 C 41/03<br>C 08 G 63/20<br>C 08 G 63/46 |
| A,D | EP-A-0 013 026 (HENKEL) * Patentansprüche * | 1-11 | |
| A | DE-A-2 536 597 (BASF) * Patentansprüche * | 1-11 | |
| A | DE-A-2 711 002 (HENKEL) * Patentansprüche * | 1-11 | |

RECHERCHIERTE SACHGEBIETE (Int. Cl. 3)

C 07 C 41/00
C 07 C 43/00

Der vorliegende Recherchenbericht wurde für alle Patentansprüche erstellt.

| Recherchenort | Abschlußdatum der Recherche | Prüfer |
|---|---|---|
| DEN HAAG | 26-03-1984 | WRIGHT M.W. |

KATEGORIE DER GENANNTEN DOKUMENTEN
X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder
    nach dem Anmeldedatum veröffentlicht worden ist
D : in der Anmeldung angeführtes Dokument
L : aus andern Gründen angeführtes Dokument
.......................................................................
& : Mitglied der gleichen Patentfamilie, überein-
    stimmendes Dokument

EPA Form 1503. 03.82